# EUROPEAN PATENT SPECIFICATION

(11) **EP 2 582 663 B1**
(45) Date of publication and mention of the grant of the patent: **12.03.2014**
(21) Application number: 11721783.6
(22) Date of filing: 27.05.2011
(51) Int. Cl.: C07C 273/04, B01J 10/00, B01D 53/94

(54) **METHOD FOR REVAMPING A SELF-STRIPPING UREA PLANT AND PROCESS FOR SYNTHESIS OF UREA**
VERFAHREN ZUR MODERNISIERUNG EINER SELBST-STRIPPENDEN HARNSTOFFANLAGE SOWIE VERFAHREN ZUR HERSTELLUNG VON HARNSTOFF
PROCÉDÉ DE REMANIEMENT D'UNE INSTALLATION DE PRODUCTION D'URÉE À STRIPAGE AUTONOME AINSI QUE PROCÉDÉ DE SYNTHÈSE D'URÉE

(30) Priority: 16.06.2010 EP 10166224
(43) Date of publication of application: 24.04.2013
(73) Proprietor: Urea Casale SA, 6900 Lugano-Besso (CH)
(72) Inventor: SCOTTO, Andrea, 6932 Breganzona (CH); GABBIADINI, Serena, 20125 Milano (IT); BERTINI, Paolo, 6900 Lugano (CH)
(74) Representative: Zardi, Marco
(86) International application number: PCT/EP2011/058741
(87) International publication number: WO 2011/157530

(56) References cited:
- EP-A1- 1 918 273
- GB-A- 1 542 371

## Description

### Field of the invention

The present invention refers to a method for revamping of a self-stripping urea plant. The invention also relates to a novel self-stripping process for synthesis of urea and related plant.

### Prior Art

A disclosure of the self-stripping or thermal-stripping process and related plant can be found e.g. in GB 1 542 371. The process is also referred to by the name of its developer Snamprogetti. A description is also found in Kirk-Othmer, Encyclopedia of Chemical Technology, 3rd Ed, Vol 23, p. 548-562.

A self-stripping urea plant comprises: a high-pressure loop, usually operating at about 140 - 160 bar; a medium-pressure (MP) section and a low-pressure (LP) recovery section where urea solution delivered by the high-pressure loop is treated to decompose carbamate and recycle ammonia. As an indication, the MP section may operate around 10 - 20 bar and the LP section may operate at a few (e.g. 3-4) bar pressure. Common values are for example 150 - 17 - 3.5 bar for high, medium and low-pressure section, respectively.

The high-pressure loop typically comprises a synthesis reactor, a steam-heated stripper, and a horizontal kettle condenser. All these items operate substantially at the same pressure.

Ammonia and CO₂ are reacted in said synthesis reactor obtaining a urea aqueous solution, comprising ammonia and unconverted ammonium carbamate; said solution is heated in the high-pressure stripper to decompose the carbamate and recover ammonia; a vapour phase containing ammonia and CO₂ produced in the stripper is condensed in the high-pressure condenser, and recycled to said reactor. The stripping process takes place with the help of heat furnished e.g. by hot steam, and without addition of an external stripping medium such as carbon dioxide (thermal stripping), or using part of the ammonia available in the solution, as a stripping agent (ammonia stripping).

In this description, the term self-stripping urea plant is used to mean a urea plant comprising a high-pressure thermal or ammonia stripping section.

There is a strong interest in the revamping of existing self-stripping urea plants. When an existing plant is revamped, one should try to maintain, whenever possible, the available equipments, for economical reasons. The applicant has noted that a major bottleneck is the capacity of the medium- and low-pressure sections. For example, when the high-pressure loop is boosted, a greater flow rate of urea solution is discharged to the downstream MP/LP sections, which means a considerably larger quantity of carbamate to decompose and ammonia to recover. The available components of said MP and LP sections, including decomposers, condensers, etc... are usually close to their maximum capacity and are not able to comply with this increased duty. Hence, the bottleneck is actually found in the MP and LP sections. Revamping also the MP and/or the LP section, together with the high-pressure section, would involve the addition or replacement of several pressure vessels and then would involve a considerable cost that may render the overall intervention less attractive.

### Summary of the invention

The invention is aimed to solve the above drawback and to provide an efficient solution for boosting a self-stripping urea plant. The idea underlying the invention is to add a further pressure level to the processing of the urea solution delivered by the HP section or by the reactor. A further step of carbamate decomposition and carbamate recovery is added upstream the MP section, i.e. at a pressure greater than the original medium pressure of the plant. According to different embodiments of the invention, said further step of carbamate decomposition may receive a portion of the urea solution delivered by the reactor and/or a portion or the full amount of the concentrated urea solution obtained from high-pressure stripping.

Hence it is proposed a new method for modifying a self-stripping urea plant, said plant comprising a high pressure urea synthesis section which includes at least a reactor, a thermal or ammonia stripper and a condenser, a medium pressure treatment section and a low pressure recovery section, the method being characterized by the following steps:
- an intermediate section is installed and connected between the high-pressure synthesis section and the medium-pressure section;
- a stream of urea solution, which is produced in the high pressure section, is directed to said intermediate section;
- a flow of concentrated solution from said intermediate section is directed to said medium pressure section;
- said intermediate section being suitable to decompose the carbamate and recover carbamate contained in said urea solution at an intermediate pressure which is lower than working pressure of said synthesis section and greater than the working pressure of said medium pressure section, obtaining said flow of concentrated solution at said intermediate pressure.

The added intermediate section comprises preferably at least a decomposition section and a recovery section, realized e.g. with a decomposer and a condenser, respectively. The steps of redirecting the urea solution to the intermediate section, and directing the concentrated solution to the downstream MP section, are carried out by providing the necessary piping, valves, pumps and auxiliary equipments, according to a per se known technique.

According to some embodiments, a stream of urea solution, which is delivered by the high pressure section and originally directed to the medium pressure section, is at least partly redirected to said intermediate section. In preferred embodiments, the full flow of urea solution delivered by the high pressure section is redirected to the newly installed intermediate section.

According to other embodiments, a stream of urea solution, which is delivered by said reactor and originally directed to said high-pressure thermal or ammonia stripper, is partly redirected to said intermediate section.

The above embodiments may be combined, i.e. the invention encompasses embodiments where a urea solution as delivered by the reactor and a more concentrated urea solution delivered by the high-pressure stripper are both directed to said intermediate section. Eventually said urea solution and concentrated (stripped) urea solution can be mixed before entering the intermediate section. In further embodiments of the invention, the intermediate section is paralleled to the high-pressure stripping section. For example, the urea solution delivered by the reactor is split into two fractions; a first fraction is directed to a high-pressure stripper, and a second fraction is directed to said intermediate section.

In some embodiments, the capacity of the high-pressure synthesis section can be increased. This increase of capacity may be achieved by modifying any of the components of the synthesis section such as reactor, stripper and condenser, or by adding further equipments to the synthesis section.

The nominal pressure of said intermediate section is preferably in the range 30 to 100 bar, more preferably 70 to 80 bar. In a preferred embodiment, the equipments of the intermediate section include at least a decomposer, a condenser and a rectification column.

The advantage of the invention is that the MP and LP section are de-bottlenecked by redirecting at least a part of the urea solution coming from the high-pressure stripper, or coming from the reactor, to the added intermediate section. If the synthesis section is boosted, the capacity of said synthesis section can be consistently increased while, at the same time, the duty of the downstream MP and LP sections is maintained substantially unaltered, since the extra duty is accomplished by the newly-installed intermediate section. In other words, the invention allows to boost the HP section in a significant manner and to debottleneck the downstream MP and LP section by the addition of the intermediate section. Installing an intermediate section in parallel with the high-pressure stripper, in accordance with some embodiments of the invention, has the advantage that the processing of the urea solution effluent from the reactor is fractioned between the HP stripper and the new intermediate section. This advantage is useful for example in cases where the capacity of the reactor is boosted, or where the HP stripper is the bottleneck of the plant, or where another or a further reactor is installed.

The modified plant operates, in practice, with a modified self-stripping process with a further pressure level, compared to the conventional process. Said process is a further aspect of the invention. Another aspect of the invention is a urea plant natively designed to operate according to the new process, as set out in the attached claims.

More in detail, an aspect of the invention is a self-stripping process for synthesis of urea, including high-pressure reaction, high-pressure thermal or ammonia stripping, and medium pressure and low pressure recovery, the process being characterized in that: a stream of urea solution, which comprises urea solution delivered by the reactor and/or concentrated urea solution delivered by said thermal or ammonia stripping, is subject to a process step of decomposition at an intermediate pressure, said intermediate pressure being lower than said high pressure and greater than said medium pressure, thus obtaining a concentrated solution at intermediate pressure, and in that said concentrated solution at intermediate pressure is directed to further treatment at said medium pressure.

A related urea plant comprises: a high-pressure section including at least a urea reactor, a thermal or ammonia stripper and a condenser; a medium-pressure urea recovery section and a low-pressure urea recovery section; an intermediate section which is installed and connected between said high-pressure section and said medium-pressure section, said intermediate section comprising at least a decomposition section and a recovery section operating at an intermediate pressure which is lower than working pressure of said synthesis section and greater than the working pressure of said medium pressure section, said intermediate section being connected to the high-pressure and medium pressure sections so that said decomposition section receives a stream of urea solution which comprises urea solution delivered by said reactor and/or concentrated urea solution delivered by said thermal or ammonia stripper, and a concentrated solution delivered by said intermediate decomposition section is directed to said medium pressure section.

In all the embodiments of the invention the high-pressure section may include one or more reactor(s), stripper(s) or condenser(s).

Further characteristics and advantages of the invention shall become clearer from the following description of some example embodiments, with reference to the attached drawings.

### Brief description of the drawings

Fig. 1 is a scheme of a conventional self-stripping plant.
Fig. 2 is a scheme of the plant of Fig. 1 modified according to a first embodiment of the invention.
Fig. 3 is a scheme of a second embodiment of the invention.
Fig. 4 is a scheme of a third embodiment of the invention.
Fig. 5 is a scheme of another embodiment of the invention.
Fig. 6 is a scheme of a further variant of the invention.

### Detailed description of preferred embodiments

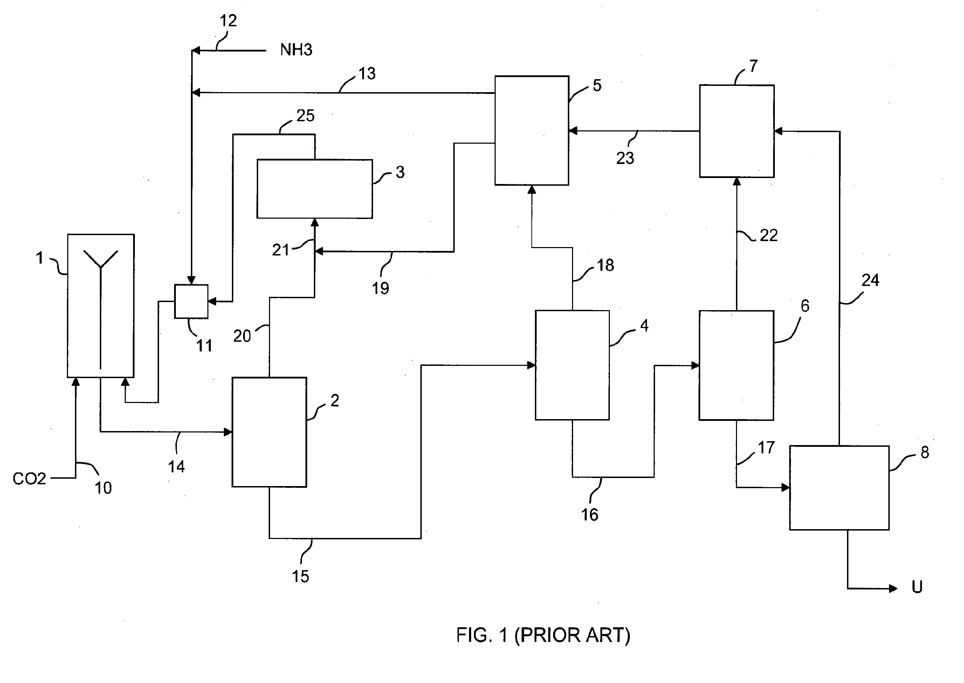

Referring to the conventional scheme of Fig. 1, the high-pressure (HP) section of the plant comprises a reactor 1, a thermal stripper or ammonia stripper 2, and a condenser 3. The medium pressure (MP) section of the plant comprises a decomposition section 4 and an ammonia recovery section, which is indicated as block 5. The low pressure (LP) section comprises a decomposition section 6 and a low pressure recovery section 7. Block 8 designates a vacuum concentration section.

A carbon dioxide feed is sent to the synthesis reactor 1 via line 10. An ejector 11 feeds the reactor 1 with fresh ammonia 12 and ammonia 13 recovered from the MP and LP sections. A urea solution 14 comprising urea, unconverted carbamate and free ammonia is discharged from the reactor 1. In some embodiments the fresh ammonia 12 may be fed to the recovery section 5, so that line 13 contains fresh and recovered ammonia.

The urea solution 14 is sent to the stripper 2, where said solution 14 is decomposed with the help of heat. A concentrated urea solution 15, produced in the stripper 2 and containing residual carbamate and ammonia, is fed to MP decomposition section 4; a further concentrated urea solution 16 is fed to the LP decomposition section 6. The solution 17 delivered by the LP section is further treated in the vacuum section 8, obtaining the urea product U.

A vapour phase 18 separated in the MP decomposition section 4, and containing mainly CO₂, ammonia and water, is treated in the ammonia recovery section 5, which delivers a liquid ammonia stream 13 and a solution 19 containing residual carbamate. To this purpose, said ammonia recovery section 5 may comprise for example a rectifying column where excess ammonia in the stream 18 is separated, and an ammonia condenser where the liquid ammonia stream 13 is produced. Bottom liquid from said rectifying column forms the carbamate solution 19.

Said carbamate solution 19 is mixed with overhead vapors 20 released by the HP stripper 2; the resulting mixed stream 21 is condensed in the high-pressure condenser 3, obtaining a condensate stream which is returned to the reactor 1 via the ejector 11.

A vapour phase 22 separated in the LP decomposing section 6 is condensed in the LP recovery section 7, obtaining ammonia solution 23 which is pumped to the MP ammonia recovery section 5, or eventually mixed with the vapour stream 18. Further ammonia is recycled via line 24 from the vacuum section 8 to the LP section of the plant.

The non-condensable gases in the output stream 25 of the condenser 3 are removed in a separator (not shown) which is located upstream the ejector 11.

The stripper 2 is typically a tube-bundle, steam-heated exchanger. The urea solution 14 from reactor 1 forms a liquid film inside the tubes of the tube bundle, whereas hot steam flowing outside the tubes supplies the heat necessary to decompose the carbamate contained in the solution. Ammonia and carbon dioxide are recovered in gaseous phase 20 at the top of the stripper. The condenser 3 is usually a horizontal shell-and-tube kettle unit, where condensation is effected on the tube side, and the condensation heat is used to produce steam.

The above details are known to a skilled person, and no further described.

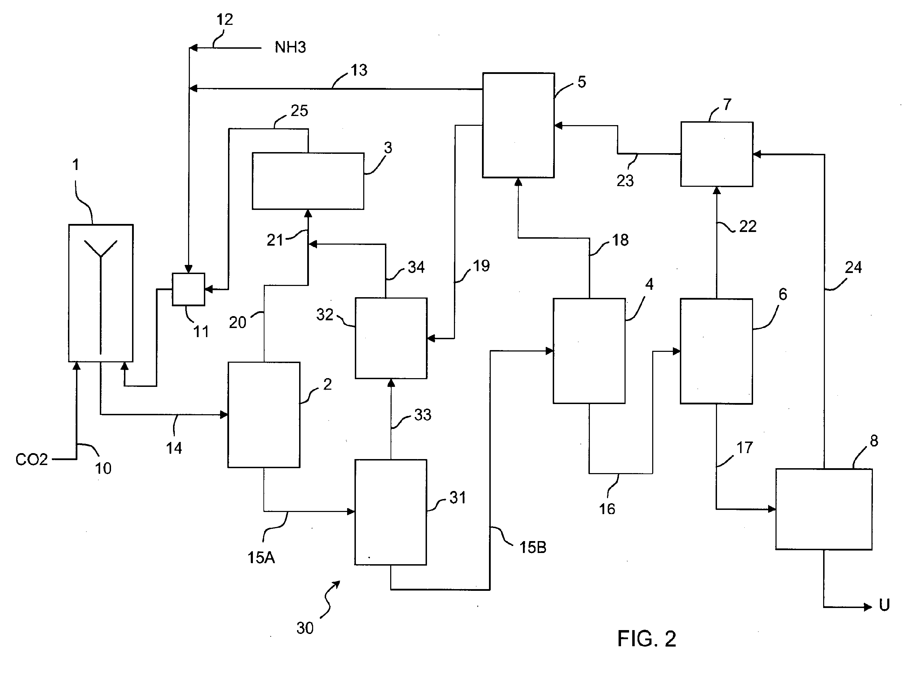

Fig. 2 discloses a scheme of the plant modified according to the invention. The urea solution from the stripper 2 is now indicated as stream 15A, which may be larger than original stream 15 in embodiments where the capacity of the high pressure synthesis section is increased.

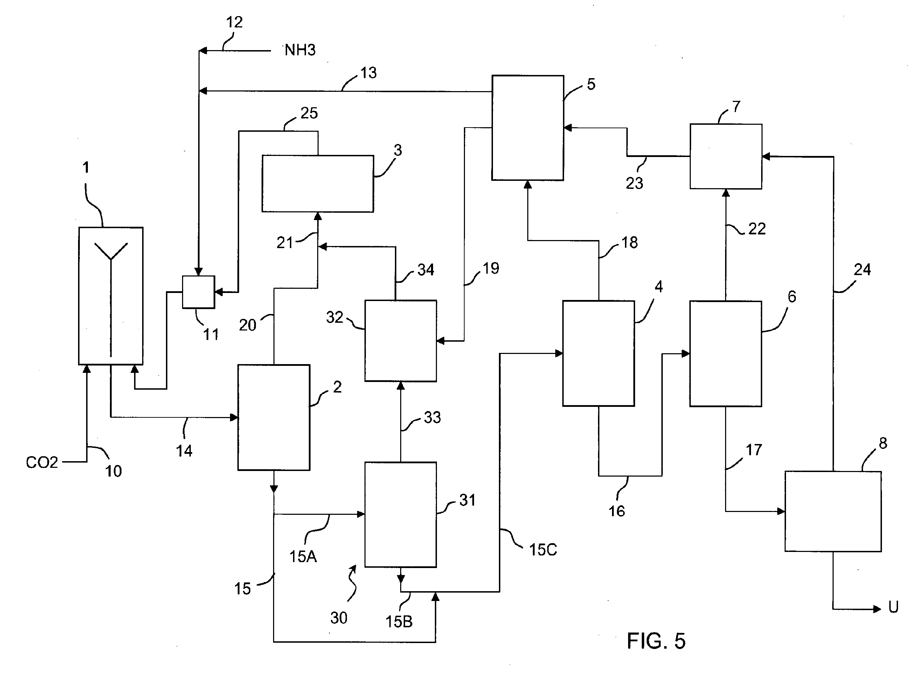

The concentrated solution from the HP stripper 2, namely the urea solution 15A, which is originally directed to the MP decomposing section 4, is now redirected to a newly-installed intermediate section 30, comprising a decomposing section 31 and a recovery section 32. In Fig. 2 the full output of urea solution, coming from the boosted high pressure section 1, is redirected to said intermediate section 30, although other embodiments of the invention provide that a portion of the urea solution is redirected to the new section 30, and a remaining portion passes from stripper 2 to the MP section (Fig. 5).

The working pressure of said intermediate section 30 is between the pressure of the high-pressure section and that of MP section, for example 30 to 100 bar and more preferably 70 to 80 bar. For example the pressure of reactor 1, stripper 2 and condenser 3 is greater than 100 bar, the medium pressure is in the range 10 to 20 bar, the low pressure is less than 5 bar. Equipments of a section operating at a certain pressure, such as reactor 1, stripper 2 and condenser 3, operate substantially all at the same pressure, with small differences that may be due to pressure losses in the connections, valves, etc...

The decomposition section 31 comprises at least a decomposer and operates substantially in the same way as the MP decomposition section 4, producing the concentrated solution 15B and a vapour phase 33 containing CO2, ammonia and water, which is condensed in the recovery section 32. The liquid carbamate solution 19 from the MP ammonia recovery section 5 is deviated and pumped to said intermediate recovery section 32, to provide condensation of said vapour phase 33. The condensate 34 is a carbamate solution containing ammonia; this solution 34 is then directed to the condenser 3 (as shown) or, if appropriate, to the reactor 1. Directing some liquid carbamate solution to the reactor instead of condenser may be appropriate in order to avoid excessive liquid amount in the ejector 11.

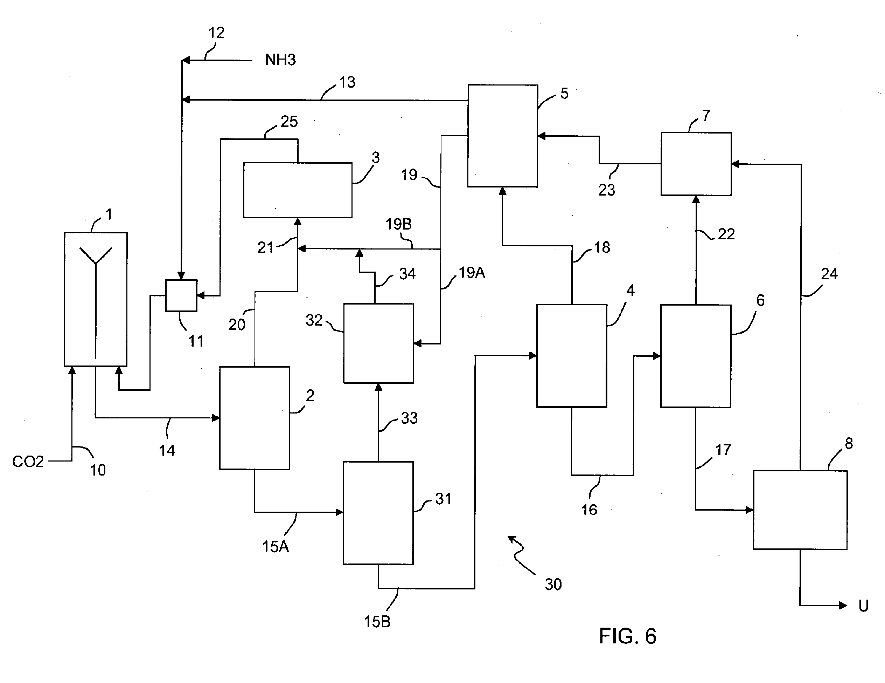

In some embodiments the flow of said carbamate solution 19 is split into two parts. A first part is directed to the intermediate recovery section 32 and the remaining part is directed to the condenser or reactor, preferably being mixed with the carbamate solution 34. Fig. 6 is an example of one of such embodiments, where a portion 19A of the carbamate solution 19 delivered by the MP recovery section 5 is deviated to the new recovery section 32. The condensate 34 is mixed with the remaining fraction 19B of said carbamate solution 19, and the mixture is then directed to the condenser or to the reactor.

Oxygen which is introduced for passivation is preferably passed also through the new section 30. For example a gaseous current containing oxygen (not shown) is usually directed from condenser 3 to the MP decomposition section 4 and finally vented from the ammonia recovery section 5; in the modified plant this current will be directed from the condenser 3 to decomposition section 31, then from decomposition section 31 to recovery section 32, and again to MP sections 4 and 5.

The carbamate solution 34 is available at the pressure of the intermediate section 30 (e.g. 70 bar) and must be elevated at the higher pressure of the condenser 3 or reactor 1. To this purpose, an option of the invention is to use high-pressure pumps that are already available on site and were originally used to pump the stream 19 from MP recovery section 5 to the condenser 3. Said pumps could require slight adaptation due to higher suction pressure but their use may help reducing costs. New pumps will be installed to raise the pressure of the solution from medium to intermediate level (e.g. from 20 bar to 70 bar): these pumps are however less expensive than those adapted to reach the higher pressure of the synthesis loop.

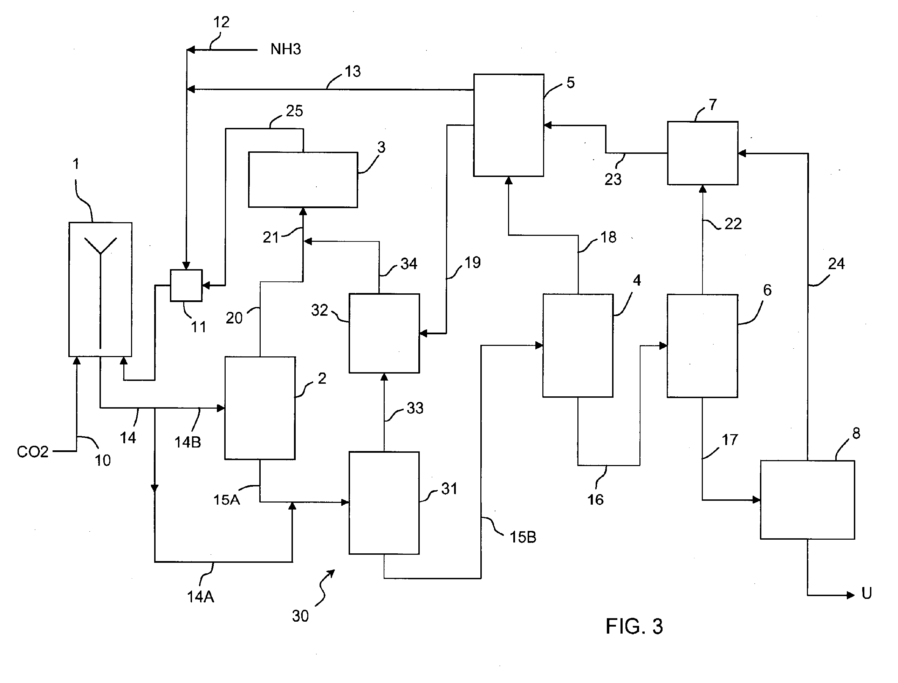

Fig. 3 illustrates an embodiment where a portion of the urea solution delivered by the reactor is sent directly to intermediate-pressure decomposition, bypassing the high-pressure stripping. Urea solution effluent 14 from the reactor 1 is divided into two portions. A first portion 14A bypasses the HP stripper 2 and is directed to the intermediate section 30, e.g. to decomposition section 31. Said first portion 14A of the urea solution can be merged with the stripped solution 15A before entering the section 31, or can be sent separately to section 31, these two variants being equally applicable. A remaining portion 14B of the reactor effluent 14 is sent to the HP stripper 2. The concentrated solution 15B delivered by said decomposition section 31 is then directed to the MP section 4.

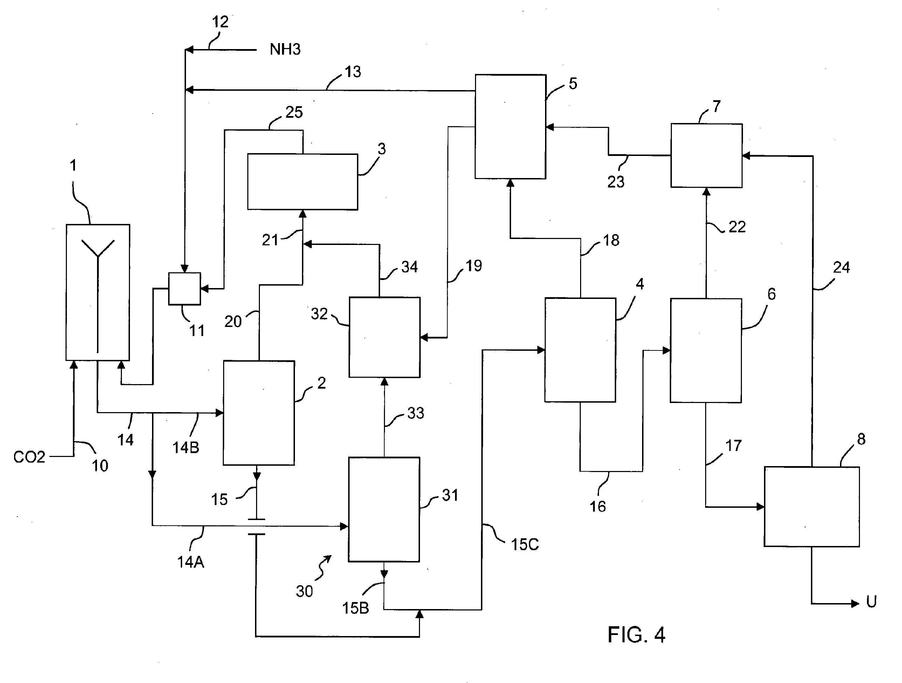

Fig. 4 illustrates an embodiment where the high-pressure stripping section and the intermediate-pressure section operate substantially in parallel. A first part 14A of the urea solution 14 is directed to the intermediate-pressure section 30; a second part 14B of said urea solution 14 is directed to the HP stripper 2. The high-pressure concentrated solution 15 and the intermediate-pressure concentrated solution 15B delivered by said section 30 are merged to form a stream 15C directed to the MP section 4.

Fig. 5 illustrates a further embodiment where the urea solution 15A directed to the intermediate section 30 is a portion of the concentrated urea solution coming from the HP stripper 2; a remaining portion 15 of said urea solution passes from stripper 2 to the MP section 4. As an example, said remaining portion 15 is merged with the concentrated urea solution 15B coming from the section 31. The embodiment of Fig. 5 is substantially a variant of the embodiment of Fig. 2; the same variant is also applicable to other embodiments of the invention such as those of Figs. 3 or Fig. 4.

Expansion valves and pumps/compressors are not shown. As apparent to a skilled reader, any stream passing from an item at a higher pressure to an item operating at a lower pressure will be suitably expanded. Vice versa, streams passing to a higher pressure will need compression / pumping. It should be noted that schemes of Figs. 1 to 6 are simplified block schemes and auxiliary items, as well as compressors, pumps and valves, are not shown.

## Claims

1. A method for revamping a self-stripping urea plant, said plant comprising a high pressure urea synthesis section which includes at least a reactor, a thermal or ammonia stripper and a condenser, a medium pressure treatment section and a low pressure recovery section, the method being **characterized by** the following steps:
- an intermediate recovery section (30) is installed and connected between the high-pressure synthesis section and the medium-pressure section;
- a stream of urea solution (14A, 15A), which is produced in the high pressure section, is directed to said intermediate section (30);
- a flow of concentrated solution (15B) from said intermediate section is directed to said medium pressure section;
- said intermediate recovery section (30) being suitable to decompose the carbamate and recover a carbamate solution from said stream of urea solution, operating at an intermediate pressure which is lower than working pressure of said synthesis section and greater than the working pressure of said medium pressure section, obtaining said flow of concentrated solution (15B) at said intermediate pressure.

2. A method according to claim 1, where a stream of urea solution (15A), which is delivered by the high pressure section and originally directed to the medium pressure section, is at least partly redirected to said intermediate section (30).

3. A method according to claim 2, said stream of urea solution (15A) being fully redirected to said intermediate section (30).

4. A method according to any of claims 1 to 3, where a stream of urea solution (14A), which is delivered by said reactor and originally directed to said high-pressure thermal or ammonia stripper, is partly redirected to said intermediate section (30).

5. A method according to any of claims 1 to 3, comprising the steps of installing at least a decomposition section (31) and a recovery section (32) to provide said intermediate section (30).

6. A method according to any of claims 1 to 4, comprising the steps of deviating a medium pressure liquid stream (19) containing recycled carbamate, obtained in said medium pressure section, or deviating at least a portion (19A) of said stream, to said recovery section (32) of the intermediate section (30), and directing a carbamate solution (34) delivered by said recovery section to the high pressure section and preferably to the high pressure condenser (3).

7. A method according to any of claims 1 to 4, comprising the step of boosting the capacity of the high-pressure synthesis section of the plant.

8. A method according to any of claims 1 to 5, said intermediate section being suitable to operate at a pressure in the range 30 to 100 bar.

9. A self-stripping process for synthesis of urea, where ammonia and carbon dioxide are reacted at a given high pressure in at least one reactor (1) to produce urea; a urea solution (14) produced in said reactor is subject to a thermal or ammonia stripping, a vapour phase (20) containing ammonia and carbon dioxide separated by said stripping is subject to condensation and recycled to said reactor, said stripping and condensation taking place substantially at said high pressure of reaction, and where urea solution is further concentrated and reactants are recovered at a medium pressure and at a low pressure, in respective medium-pressure and low-pressure recovery sections, the process being **characterized in that**:
- a stream (14A, 15A) of urea solution, which comprises urea solution (14A) delivered by said reactor and/or concentrated urea solution (15A) delivered by said thermal or ammonia stripping, is subject to a process step of decomposition (31) at an intermediate pressure, said intermediate pressure being lower than said high pressure and greater than said medium pressure, thus obtaining a concentrated solution (15B) at intermediate pressure, and
- said concentrated solution (15B) at intermediate pressure is directed to further treatment at said medium pressure.

10. A process according to claim 9, a vapour phase (33) obtained from said decomposition at intermediate pressure being condensed to obtain a carbamate solution (34) at intermediate pressure, said liquid stream being fed to said high-pressure section.

11. A process according to claim 9 or 10, the high pressure being greater than 100 bar, the medium pressure being in the range 10 to 20 bar, the low pressure being less than 5 bar, and said intermediate pressure being 30 to 100 bar.

12. A urea plant operating according to the self-stripping process, comprising a high-pressure section including a urea reactor (1), a thermal or ammonia stripper (2) and a condenser (3), and also comprising a medium-pressure urea recovery section (4, 5) and a low-pressure urea recovery section (6, 7), the plant being **characterized by** comprising an intermediate section (30) which is installed and connected between said high-pressure section and said medium-pressure section, said intermediate section comprising at least a decomposition section (31) and a recovery section (32) operating at an intermediate pressure which is lower than working pressure of said synthesis section and greater than the working pressure of said medium pressure section, and said intermediate section (30) being connected to the high-pressure and medium pressure sections so that the decomposition section (31) of said intermediate section receives a stream (14A, 15A) of urea solution, which comprises urea solution (14A) delivered by said reactor and/or concentrated urea solution (15A) delivered by said thermal or ammonia stripping, and wherein a concentrated solution (15B) delivered by said intermediate decomposition section is directed to said medium pressure section.

13. A plant according to claim 12, said recovery section (32) being connected to receive a portion (19A) or the total amount of a medium pressure liquid stream (19) containing recycled carbamate, obtained in said medium pressure section, and to deliver a carbamate solution (34) to the high pressure section and preferably to the condenser (3) of said high pressure section.

## Patentansprüche

1. Ein Verfahren zum Modernisieren einer selbststrippenden Harnstoffanlage, wobei die Anlage umfasst einen Hochdruckharnstoffsyntheseabschnitt, welcher zumindest einen Reaktor, einen Thermal- oder Ammoniakstripper und einen Kondensator aufweist, einen Mitteldruckbehandlungsabschnitt und einen Niederdruckrückgewinnungsabschnitt, wobei das Verfahren sich durch die folgenden Schritte auszeichnet:
- ein Zwischenrückgewinnungsabschnitt (30) ist zwischen dem Hochdrucksyntheseabschnitt und dem Mitteldruckabschnitt angebracht und angeschlossen;
- ein Strom an Harnstofflösung (14A, 15A), welcher in dem Hochdruckabschnitt erzeugt wird, wird zu dem Zwischenabschnitt (30) geleitet;
- ein Strom an konzentrierter Lösung (15B) wird von dem Zwischenabschnitt zu dem Mitteldruckabschnitt geleitet;
- wobei der Zwischenrückgewinnungsabschnitt (30) geeignet ist, um Carbamat zu zersetzen und eine Carbamatlösung aus dem Strom an Harnstofflösung rüchzugewinnen, unter einem Zwischendruck operiert, welcher niedriger ist als der Arbeitsdruck des Syntheseabschnitts und höher als der Arbeitsdruck des Mitteldruckabschnitts, und den Strom an konzentrierter Lösung (15B) unter Zwischendruck erzielt.

2. Verfahren nach Anspruch 1, wo ein Strom an Harnstofflösung (15A), welcher von dem Hochdruckabschnitt geliefert und unbearbeitet zu dem Mitteldruckabschnitt geleitet wird, zumindest teilweise zu dem Zwischenabschnitt (30) umgeleitet wird.

3. Verfahren nach Anspruch 2, wobei der Strom an Harnstofflösung (15A) vollständig zu dem Zwischenabschnitt (30) umgeleitet wird.

4. Verfahren nach einem der Ansprüche 1 bis 3, wo ein Strom an Harnstofflösung (14A), welcher von dem Reaktor geliefert und unbearbeitet zu dem Hochdruckthermal- oder -ammoniakstripper geleitet wird, zumindest teilweise zu dem Zwischenabschnitt (30) umgeleitet wird.

5. Verfahren nach einem der Ansprüche 1 bis 3, umfassend die Schritte des Anbringens von zumindest einem Zersetzungsabschnitt (31) und einem Rückgewinnungsabschnitt (32), um den Zwischenabschnitt bereit zu stellen.

6. Verfahren nach einem der Ansprüche 1 bis 4, umfassend die Schritte des Umlenkens eines Mitteldruckflüssigkeitsstroms (19), welcher wieder aufbereitetes Carbamat enthält, das in dem Mitteldruckabschnitt erzielt wird, oder des Umlenkens von zumindest eines Teils (19A) des Stroms zu dem Rückgewinnungsabschnitt (32) des Zwischenabschnitts (30), und des Leitens einer Carbamatlösung (34), welche von dem Rückgewinnungsabschnitt geliefert wird, zu dem Hochdruckabschnitt und vorzugsweise zu dem Hochdruckkondensator (3).

7. Verfahren nach einem der Ansprüche 1 bis 4, umfassend den Schritt des Erhöhens der Kapazität des Hochdrucksyntheseabschnitts der Anlage.

8. Verfahren nach einem der Ansprüche 1 bis 5, wobei der Zwischenabschnitt geeignet ist, um unter einem Druck im Bereich von 30 bis 100 bar zu arbeiten.

9. Ein selbststrippender Prozess für die Synthese von Harnstoff, wo Ammoniak und Kohlenstoffdioxid unter einem vorgegebenen Hochdruck in zumindest einem Reaktor (1) umgesetzt werden, um Harnstoff zu erzeugen; wobei eine Harnstofflösung (14), welche in dem Reaktor erzeugt wird, einer Thermal- oder Ammoniakstrippung unterliegt, und eine Dampfphase (20), welche Ammoniak und Kohlenstoffdioxid enthält, das von der Strippung zerlegt wird, einer Kondensation unterliegt und in dem Reaktor wieder aufbereitet wird, wobei die Strippung und die Kondensation im Wesentlichen unter Hochdruck der Reaktion stattfinden, und wo die Harnstofflösung weiterhin konzentriert wird und die Reaktionspartner unter einem Mitteldruck und unter einem Niederdruck rückgewonnen werden, in den jeweiligen Mitteldruck- und Niederdruckrückgewinnungsabschnitten, wobei der Prozess **dadurch gekennzeichnet ist, dass**:
- ein Strom (14A, 15A) an Harnstofflösung, welcher Harnstofflösung (14A), die von dem Reaktor geliefert wird, und/oder konzentrierte Harnstofflösung (15A), die von der Thermal- oder Ammoniakstrippung geliefert wird, umfasst, einem Prozessschritt des Zersetzens (31) unter einem Zwischendruck unterliegt, wobei der Zwischendruck niedriger ist als der Hochdruck und höher als der Mitteldruck, um somit eine konzentrierte Lösung (15B) unter Zwischendruck zu erzielen, und
- wobei die konzentrierte Lösung (15B) unter Zwischendruck zu einer weiteren Behandlung unter Mitteldruck geleitet wird.

10. Verfahren nach Anspruch 9, wobei eine Dampfphase (33), welche von dem Zersetzen unter Zwischendruck erzielt wird, verdichtet wird, um eine Carbamatlösung (34) unter Zwischendruck zu erzielen, wobei der Flüssigkeitsstrom dem Hochdruckabschnitt zugeführt wird.

11. Verfahren nach Anspruch 9 oder 10, wobei der Hochdruck höher ist als 100 bar, der Mitteldruck in dem Bereich von 10 bis 20 bar liegt, der Niederdruck niedriger ist als 5 bar, und wobei der Zwischendruck 30 bis 100 bar beträgt.

12. Eine Harnstoffanlage gemäß dem selbststrippenden Prozess, umfassend einen Hochdruckabschnitt, welcher einen Harnstoffreaktor (1), einen Thermal- oder Ammoniakstripper (2) und einen Kondensator (3) aufweist, und ebenso umfassend einen Mitteldruckharnstoffrückgewinnungsabschnitt (4, 5) und einen Niederdruckharnstoffrückgewinnungsabschnitt (6, 7), wobei die Anlage **gekennzeichnet ist durch** das Umfassen eines Zwischenabschnitts (30), welcher zwischen dem Hochdruckabschnitt und dem Mitteldruckabschnitt angebracht und angeschlossen ist, wobei der Zwischenabschnitt zumindest einen Zersetzungsabschnitt (31) und einen Rückgewinnungsabschnitt (32) umfasst, welcher unter einem Zwischendruck arbeitet, der niedriger ist als der Arbeitsdruck des Syntheseabschnitts und höher als der Arbeitsdruck des Mitteldruckabschnitts, und wobei der Zwischenabschnitt (30) an den Hochdruck- und den Mitteldruckabschnitt angeschlossen ist, sodass der Zersetzungsabschnitt (31) des Zwischenabschnitts einen Strom (14A, 15A) an Harnstofflösung empfängt, welcher die Harnstofflösung (14A), die von dem Reaktor geliefert wird, und/oder konzentrierte Harnstofflösung (15A), die von der Thermal- oder Ammoniakstrippung geliefert wird, umfasst, und wobei eine konzentrierte Lösung (15B), welche von dem Zwischenzersetzungsabschnitt geliefert wird, zu dem Mitteldruckabschnitt geleitet wird.

13. Anlage nach Anspruch 12, wobei der Rückgewinnungsabschnitt (32) angeschlossen wird, um einen Teil (19A) oder die vollständige Menge eines Mitteldruckflüssigkeitsstrom (19) zu empfangen, welcher wieder aufbereitetes Carbamat enthält, das in dem Mitteldruckabschnitt erzielt wird, und um eine Carbamatlösung (34) zu dem Hochdruckabschnitt und vorzugsweise zu dem Kondensator (3) des Hochdruckabschnitts zu liefern.

## Revendications

1. Procédé pour moderniser une installation de production d'urée par auto-revaporisation, ladite installation comportant un tronçon de synthèse d'urée à haute pression qui inclut au moins un réacteur, un revaporisateur d'ammoniac ou un revaporisateur thermique et un condenseur, un tronçon de traitement à moyenne pression et un tronçon de récupération à basse pression, le procédé étant **caractérisé par** les étapes suivantes :
- un tronçon de récupération intermédiaire (30) est installé et raccordé entre le tronçon de synthèse à haute pression et le tronçon à moyenne pression,
- un flux de solution d'urée (14A, 15A), qui est produit dans le tronçon à haute pression, est dirigé vers ledit tronçon intermédiaire (30),
- un écoulement de solution concentrée (15B) provenant dudit tronçon intermédiaire est dirigé vers ledit tronçon à moyenne pression,
- ledit tronçon de récupération intermédiaire (30) étant adapté pour décomposer le carbamate et récupérer une solution de carbamate à partir dudit flux de solution d'urée, fonctionnant à une pression intermédiaire qui est inférieure à la pression de service dudit tronçon de synthèse et supérieure à la pression de service dudit tronçon à moyenne pression, en obtenant ledit écoulement de solution concentrée (15B) à ladite pression intermédiaire.

2. Procédé selon la revendication 1, dans lequel un flux de solution d'urée (15A), qui est délivré par le tronçon à haute pression et initialement dirigé vers le tronçon à moyenne pression, est au moins partiellement redirigé vers ledit tronçon intermédiaire (30).

3. Procédé selon la revendication 2, ledit flux de solution d'urée (15A) étant entièrement redirigé vers ledit tronçon intermédiaire (30).

4. Procédé selon l'une quelconque des revendications 1 à 3, dans lequel un flux de solution d'urée (14A), qui est délivré par ledit réacteur et initialement dirigé vers ledit revaporisateur d'ammoniac ou ledit revaporisateur thermique à haute pression, est partiellement redirigé vers ledit tronçon intermédiaire (30).

5. Procédé selon l'une quelconque des revendications 1 à 3, comportant les étapes consistant à installer au moins un tronçon de décomposition (31) et un tronçon de récupération (32) pour fournir ledit tronçon intermédiaire (30).

6. Procédé selon l'une quelconque des revendications 1 à 4, comportant les étapes consistant à dévier un flux de liquide à moyenne pression (19) contenant du carbamate recyclé, obtenu dans ledit tronçon à moyenne pression, ou à dévier au moins une partie (19A) dudit flux, vers ledit tronçon de récupération (32) du tronçon intermédiaire (30), et diriger une solution de carbamate (34) délivrée par ledit tronçon de récupération vers ledit tronçon à haute pression et de préférence vers le condenseur à haute pression (3).

7. Procédé selon l'une quelconque des revendications 1 à 4, comportant l'étape consistant à accroître la capacité du tronçon de synthèse à haute pression de l'installation.

8. Procédé selon l'une quelconque des revendications 1 à 5, ledit tronçon intermédiaire étant adapté pour fonctionner à une pression située dans l'intervalle de 30 à 100 bars.

9. Procédé d'auto-revaporisation pour la synthèse d'urée, dans lequel de l'ammoniac et du dioxyde de carbone entrent en réaction à une haute pression donnée dans au moins un réacteur (1) pour produire de l'urée, une solution d'urée (14) produite dans ledit réacteur est soumise à une revaporisation d'ammoniac ou une revaporisation thermique, une phase vapeur (20) contenant l'ammoniac et le dioxyde de carbone séparés par ladite revaporisation est soumise à une condensation et recyclée jusqu'audit réacteur, lesdites revaporisation et condensation ayant lieu pratiquement à ladite haute pression de réaction, et dans lequel la solution d'urée est également concentrée et les réactifs sont récupérés à une moyenne pression et à une basse pression, dans des tronçons de récupération à moyenne pression et à basse pression respectifs, le procédé étant **caractérisé en ce que** :
- un flux (14A, 15A) de solution d'urée, qui comporte une solution d'urée (14A) délivrée par ledit réacteur et/ou une solution d'urée concentrée (15A) délivrée par ladite revaporisation d'ammoniac ou ladite revaporisation thermique, est soumis à une étape de procédé de décomposition (31) à une pression intermédiaire, ladite pression intermédiaire étant inférieure à ladite haute pression et supérieure à ladite moyenne pression, en obtenant ainsi une solution concentrée (15B) à une pression intermédiaire, et
- ladite solution concentrée (15B) à une pression intermédiaire est dirigée en vue d'un traitement ultérieur à ladite moyenne pression.

10. Procédé selon la revendication 9, une phase vapeur (33) obtenue à partir de ladite décomposition à pression intermédiaire étant condensée pour obtenir une solution de carbamate (34) à pression intermédiaire, ledit flux de liquide étant acheminé jusqu'audit tronçon à haute pression.

11. Procédé selon la revendication 9 ou 10, la haute pression étant supérieure à 100 bars, la moyenne pression étant dans l'intervalle de 10 à 20 bars, la basse pression étant inférieure à 5 bars, et ladite pression intermédiaire étant comprise entre 30 et 100 bars.

12. Installation de production d'urée fonctionnant selon le procédé d'auto-revaporisation, comportant un tronçon à haute pression incluant un réacteur d'urée (1), un revaporisateur d'ammoniac ou un revaporisateur thermique (2) et un condenseur (3), et comportant également un tronçon de récupération d'urée à moyenne pression (4, 5) et un tronçon de récupération d'urée à basse pression (6, 7), l'installation étant **caractérisée en ce qu'**elle comporte un tronçon intermédiaire (30) qui est installé et raccordé entre ledit tronçon à haute pression et ledit tronçon à moyenne pression, ledit tronçon intermédiaire comportant au moins un tronçon de décomposition (31) et un tronçon de récupération (32) fonctionnant à une pression intermédiaire qui est inférieure à la pression de service dudit tronçon de synthèse et supérieure à la pression de service dudit tronçon à moyenne pression, et ledit tronçon intermédiaire (30) étant raccordé au tronçon à haute pression et à moyenne pression de sorte que le tronçon de décomposition (31) dudit tronçon intermédiaire reçoit un flux (14A, 15A) de solution d'urée, qui comporte une solution d'urée (14A) délivrée par ledit réacteur et/ou une solution d'urée concentrée (15A) délivrée par ladite revaporisation d'ammoniac ou ladite revaporisation thermique, et dans lequel une solution concentrée (15B) délivrée par ledit tronçon de décomposition intermédiaire est dirigée vers ledit tronçon à moyenne pression.

13. Installation selon la revendication 12, ledit tronçon de récupération (32) étant raccordé de manière à recevoir une partie (19A) ou la quantité totale d'un flux de liquide à moyenne pression (19) contenant du carbamate recyclé, obtenu dans ledit tronçon à moyenne pression, et à délivrer une solution de carbamate (34) au tronçon à haute pression et de préférence au condenseur (3) dudit tronçon à haute pression.
